(19) Europäisches Patentamt
European Patent Office
Office européen des brevets

(11) Numéro de publication : **0 486 409 A1**

(12) **DEMANDE DE BREVET EUROPEEN**

(21) Numéro de dépôt : **91420400.3**

(22) Date de dépôt : **12.11.91**

(51) Int. Cl.[5] : **A01N 43/653,** A01N 43/50, C07D 249/08, C07D 233/60, C07D 521/00

(30) Priorité : **13.11.90 FR 9014399**

(43) Date de publication de la demande : **20.05.92 Bulletin 92/21**

(84) Etats contractants désignés : **AT BE CH DE DK ES FR GB GR IT LI LU NL SE**

(71) Demandeur : **RHONE-POULENC AGROCHIMIE
14-20, rue Pierre Baizet
F-69009 Lyon (FR)**

(72) Inventeur : **Greiner, Alfred
35, route de St Romain
F-69450 St Cyr au Mont d'Or (FR)**
Inventeur : **Hutt, Jean
200 bis, rue de St Cyr
F-69009 Lyon (FR)**
Inventeur : **Mugnier, Jacques
La Bathie
F-74330 La Balme de Lillingy (FR)**

(74) Mandataire : **Chrétien, François et al
RHONE-POULENC AGROCHIMIE- DPI B.P. 9163
F-69263 Lyon Cédex 09 (FR)**

(54) **Procédé pour protéger les produits de multiplication des végétaux à l'aide de triazolyl/ou imidazolyl-méthyl allyl alcool etcomposés nouveaux pour mettre en oeuvre le procédé.**

(57) Procédé pour protéger à titre curatif ou préventif les produits de multiplication des végétaux et les végétaux en résultant contre les maladies fongiques, caractérisé en ce que l'on applique audit produit de multiplications une composition fongicide contenant un support inerte acceptable en agriculture, éventuellement un tensio actif également acceptable en agriculture et au moins un azole de formule :

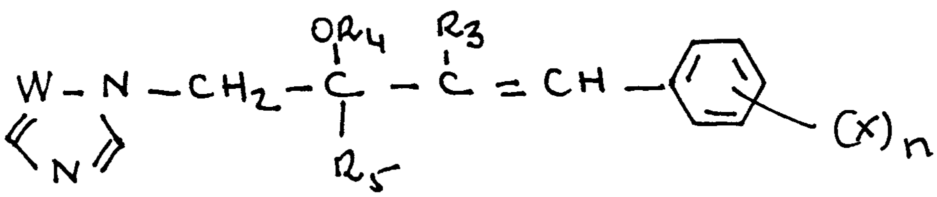

I

dans laquelle de préférence :
W est -N= ou -CH=, $R_4$ = H, X = halogène, n = 1, 2,3, $R_5$ est $C(R_1,R_2)CH_3$ ou $CH_2C(R_6,R_7)OR_6$ avec $R_6$ et $R_7$ hydrogène ou méthyle ou ensemble = O et $R_8$ est un alkyle ($C_1$-$C_3$).
$R_1$, $R_2$ sont H ou alkyle ou cycloalkyle.

L'invention concerne également les produits de multiplication des végétaux (notamment les graines) revêtus de tels composés.

EP 0 486 409 A1

Jouve, 18, rue Saint-Denis, 75001 PARIS

L'invention concerne un procédé pour protéger à titre curatif ou préventif les produits de multiplication des végétaux et les végétaux en résultant contre les maladies fongiques par application auxdits produits de multiplication ou aux végétaux d'un composé azolique.

Elle concerne également les produits de multiplication des végétaux revêtus desdits composés azoliques.

Elle concerne également les composés nouveaux pour mettre en oeuvre le procédé.

On connaît dans l'art des azolylméthyl allylalcools utiles comme fongicides foliaire notamment par EP-A-52424, EP-A-333059, EP-A-40345, FR-A-2634482, FR-A-2638454. Ces composés sont néanmoins réputés pour présenter un effet physiologique important sur les plantes (régulation de croissance, phytotoxicité).

De façon la plus inattendue qu'il soit, il a été trouvé que certains azolylméthylallyalcools déjà connus ou non décrits jusqu'à présent permettaient, en raison de leur absence de phytotoxicité, une utilisation dans le cadre de la protection des produits de multiplication des végétaux et des végétaux eux-mêmes mais encore permettaient de protéger la culture après la levée qui a lieu normalement cinq jours après le semis, éventuellement jusqu'à la récolte et avait comme intérêt industriel majeur d'éviter dans de nombreux cas un ou plusieurs traitements foliaires.

L'invention concerne donc en premier lieu un procédé pour protéger à titre curatif ou préventif les produits de multiplications des végétaux et les végétaux en résultant ou de protection des végétaux eux-mêmes contre les maladies fongiques, caractérisé en ce que l'on applique audit produit de multiplications une composition fongicide contenant un support inerte acceptable en agriculture, éventuellement un tensio actif également acceptable en agriculture et au moins un azole de formule :

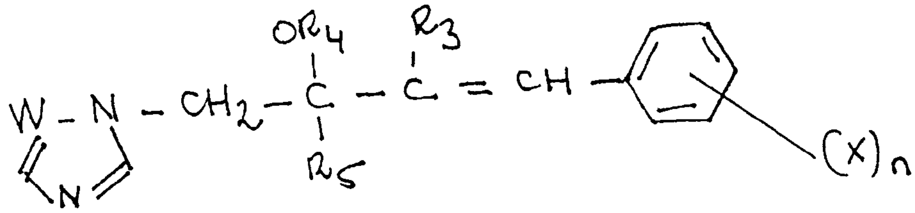

I

dans laquelle :

– W représente un groupe trivalent constitué soit d'un groupe = CH-, soit d'un atome d'azote -N = ,

– X est un atome d'halogène, notamment le chlore, le brome, le fluor, ou un groupe $C_1$-$C_4$ alkyle ou $C_1$-$C_4$ alkoxy éventuellement halogéné.

– n est un nombre entier positif ou nul, inférieur à 6, les groupements X pouvant être identiques ou différents lorsque n est plus grand que 1,

– $R_1$, $R_2$, identiques ou différents, représentent l'atome d'hydrogène ou un radical $C_1$-$C_4$ alkyle ou alkylène $C_2$-$C_4$ éventuellement substitué (par exemple par un ou plusieurs atomes ou radicaux tels que les atomes d'halogènes, les radicaux $C_1$-$C_4$ alkoxy), les radicaux $C_3$-$C_7$ cycloalkyle , $C_6$-$C_{10}$ aryle (notamment phényle), $C_7$-$C_{11}$ aralkyle (notamment benzyle), ces divers radicaux pouvant être éventuellement substitués (par exemple par un ou plusieurs atomes ou radicaux tels que les atomes d'halogènes, les radicaux $C_1$-$C_4$ alkyle, les radicaux mono ou polyhalo $C_1$-$C_4$ alkyle, les radicaux $C_1$-$C_4$ alkoxy et les radicaux mono ou polyhalo $C_1$-$C_4$ alkoxy), $R_1$, $R_2$ ensemble peuvent former une chaine $C_2$-$C_5$ hydrocarbonée constituant un cycle avec le carbone auquel $R_1$, $R_2$ sont reliés, cette chaine étant éventuellement substituée comme pour les radicaux $C_6$-$C_{10}$ aryle précédemment cités.

$R_4$ représente l'atome d'hydrogène, un radical $C_1$-$C_4$ alkyle éventuellement substitué (par exemple par un ou plusieurs atomes ou radicaux tels que les atomes d'halogènes, les radicaux $C_1$-$C_4$ alkoxy,

$R_5$ est un groupe $C(R_1,R_2)CH_3$ dans lequel $R_1$ et $R_2$ sont tels que définis ci-dessus, ou un groupe $CH_2C(R_6,R_7)$-$OR_8$ dans lequel $R_6$ et $R_7$, identiques ou différents, sont un atome d'hydrogène ou un méthyle ou peuvent former ensemble un = O et $R_8$ est un atome d'hydrogène ou un alcoyle de 1 à 3 atomes de carbone.

$R_3$ est un atome d'hydrogène ou un radical $C_1$-$C_6$ alkyle éventuellement substitué (par exemple par un ou plusieurs atomes ou radicaux tels que les atomes d'halogène, les radicaux $C_1$-$C_4$ alkoxy) ou $C_3$-$C_7$ cycloalkyle éventuellement substitué comme pour les radicaux $R_1$ ou $R_2$ ci-dessus,

$R_3$ est encore un radical $C_1$-$C_6$ alcoxy substitué éventuellement comme le radical $C_1$-$C_6$ alkyle ou un atome d'halogène (de préférence F, C1, Br) et les sels acceptables en agriculture de ces composés.

L'invention concerne également les formes salifiées des composés selon l'invention. Les formes salifiées sont les formes acceptables en agriculture parmi lesquelles on peut citer : les chlorhydrate, sulfate, oxalate, nitrate ou arylsulfonate ainsi que les complexes d'addition de ces composés avec des sels métalliques, et

notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Au sens du présent texte, on entend que lorsque aucune précision n'est donnée les radicaux concernés peuvent être ramifiés ou linéaires. Le terme éventuellement halogéné signifie éventuellement mono ou poly halogéné.

La stéréochimie de la double liaison compte tenu des contraintes stériques est telle que majoritairement le groupe phényle est en opposition (trans) par rapport au carbone qui porte le groupe azolylméthyle.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

En vue des applications fongicides selon l'invention, l'invention concerne de préférence les composés de formule I dans laquelle n = 1, 2 ou 3, et de préférence X est un halogène choisi parmi le chlore, le brome ou le fluor.

Il a été également trouvé qu'il était préférable d'utiliser les composés de formule I dans laquelle n = 1 ou 2, et X est un atome d'halogène placé en para, lorsque n = 1 et méta para ou ortho para lorsque n = 2, de préférence n = 1 et X est placé en para.

Très avantageusement X est l'atome de chlore .

Compte tenu des restrictions définies ci-dessus prises séparément ou en combinaisons il a été trouvé qu'il était préférable en raison des propriétés fongicides d'utiliser les composés de formule I dans laquelle W est -N =.

Compte tenu ou non des restrictions définies ci-dessus prises séparément ou en combinaisons il a été trouvé qu'il était préférable en raison des propriétés fongicides d'utiliser les composés de formule I dans laquelle $R_1$ et $R_2$ sont choisis parmi l'atome d'hydrogène ou les radicaux $C_1$-$C_4$ alkyle, de préférence méthyle, éthyle.

Compte tenu ou non des restrictions définies ci-dessus prises séparément ou en combinaisons il a été trouvé qu'il était préférable en raison des propriétés fongicides d'utiliser les composés où $R_4$ est l'atome d'hydrogène ou $C_1$-$C_4$ alkyle, très avantageusement $R_4$ est l'atome d'hydrogène.

Compte tenu ou non des restrictions définies ci-dessus prises séparément ou en combinaisons il a été trouvé qu'il était préférable en raison des propriétés fongicides d'utiliser les composés où $R_3$ est un atome d'hydrogène ou est un radical $C_1$-$C_4$ alkyle de préférence méthyle ou éthyle.

On préfère les composés répondant à la formule Ia:

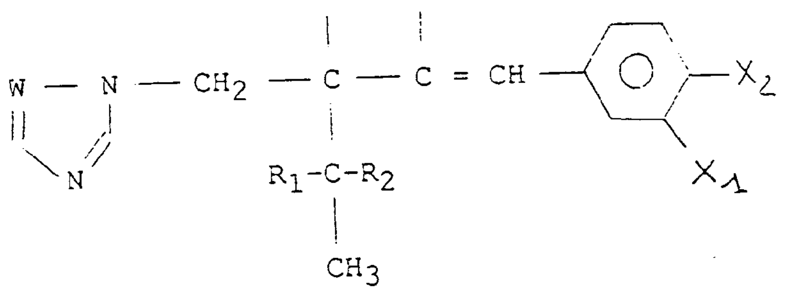

Ia

$X_2$ est Hal (de préférence Cl)

$R_3$ est l'atome d'hydrogène ou de chlore ou un radical, $C_1$-$C_4$ alkyle (de préférence méthyle, éthyle),

$R_1$, $R_2$ sont choisis parmi l'atome d'hydrogène ou les radicaux $C_1$-$C_4$ alkyle (de préférence méthyle).

De préférence les composés sont les suivants :

-1-(4-chlorophenyl)-4,4-dimethyl-3-(1,2,4-triazol-1-ylmethyl)3-ol-1-pentène.
(W = N, X = 4-Cl, $R_4$ = H, $R_3$ = H, $R_1$ = $R_2$ = $CH_3$)

-2-(4-chlorobenzylidène)-4-methyl-3-(1,2,4-triazol-1-ylmethyl)-3-pentanol.
(W = N, X = 4-Cl, $R_4$ = H, $R_1$=$CH_3$ $R_2$ = H, $R_3$ = $CH_3$)

-1-(4-chlorophenyl)-4-methyl-3-(1,2,4-triazol-1-ylmethyl)-3-ol-1-pentène.
(W = N, X = 4-Cl, $R_4$ = H, $R_3$ = H, $R_1$ = H, $R_2$ = $CH_3$)

-2-(4-chlorobenzylidène)-4,4-dimethyl-3-(1,2,4-triazol-1-ylmethyl)-3-pentanol
(W = N, X = 4-Cl, $R_4$ = H, $R_3$ = $R_1$ = $R_1$ = $CH_3$)

-1-(4-chlorophényl)-2-chloro-4-méthyl-3-(1H-1,2,4-triazoyl-1-ylméthyl-3-ol 1-pentène (W = N, X = 4-Cl, $R_4$ = H, $R_3$ = Cl, $R_1$ = $R_2$ = H)

L'invention concerne également les composés de formule I à l'exception des composés suivants :

| 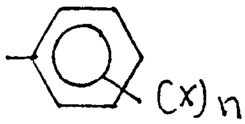 | $R_1$ | $R_2$ | $R_3$ | $R_4$ | W |
|---|---|---|---|---|---|
| 2,4-dichlorophenyl | $CH_3$ | $CH_3$ | H | H | N |
| 4-chlorophenyl | $CH_3$ | $CH_3$ | H | H | N |
| 4-chlorophenyl | $CH_3$ | $CH_3$ | H | H | CH |
| 4-fluorophenyl | $CH_3$ | $CH_3$ | H | H | N |
| 2-chlorophenyl | $CH_3$ | $CH_3$ | H | H | N |
| 2-chloro4-fluorophenyl | $CH_3$ | $CH_3$ | H | H | N |
| 2-fluoro-4chlorophenyl | $CH_3$ | $CH_3$ | H | H | N |
| 4-chlorophenyl | -1-methylcyclopropyl- | | H | H | N |
| 4-methoxyphenyl | $CH_3$ | $CH_3$ | H | H | N |
| 4-methylphenyl | $CH_3$ | $CH_3$ | H | H | N |
| 4-chlorophenyl | $CH_3$ | $CH_3$ | H | $CH_3$ | N |
| 4-fluorophényl | $CH_3$ | $CH_3$ | H | $CH_3$ | N |
| phenyl | $CH_3$ | $CH_3$ | H | H | N |
| 4-methoxyphenyl | $CH_3$ | $CH_3$ | H | $CH_3$ | N |
| 2-methylphenyl | $CH_3$ | $CH_3$ | H | H | N |
| 2-methylphenyl | $CH_3$ | $CH_3$ | H | H | CH |
| 4-fluorophenyl | $CH_3$ | $CH_3$ | H | H | CH |
| 4-methylphenyl | $CH_3$ | $CH_3$ | H | H | CH |
| 2,6-dichlorophenyl | $CH_3$ | $CH_3$ | H | H | CH |

Par le terme "produit de multiplication", on entend désigner toutes les parties génératives de la plante qu'on peut utiliser pour la multiplication de celle-ci. On citera par exemple les graines (semences au sens étroit), les racines, les fruits, les tubercules, les bulbes, les rhizomes, les parties de plantes. On mentionnera également les plantes germées et les jeunes plants qui doivent être transplantés après germination ou après la sortie de terre. Ces jeunes plants peuvent être protégés avant la transplantation par un traitement total ou partiel par immersion.

On préferera les graines pour les cultures autres que la pomme de terre.

Parmi les produits de multiplication convenant pour le procédé de traitement selon l'invention on préfèrera les :

– graines de dicotyledones : pois, concombre, melon, soja, coton, tournesol, colza, haricot, lin, betterave
– graines de monocotylédones : céréales (blé, orge, seigle, avoine), maïs, riz
– ou les tubercules de pomme de terre.

De préférence les graines seront revêtues de 0,1 à 500 g de matière active par quintal de graine, de préférence 1 à 400 g par quintal.

De préférence, dans le cas des tubercules, celles-ci sont revêtues d'une quantité de matière active correspondant au trempage dudit produit dans une composition contenant 0,1g/l à 100 g/l de matière active.

Les compositions selon l'invention contiennent habituellement entre 0,5 et 95 % de matière active.

Par le terme "support", dans le présent exposé, on désigne une matière organique ou minérale, naturelle ou synthétique, avec laquelle la matière active est associée pour faciliter son application sur la plante, sur des

graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc ...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est souvent requise parce que la matière active et/ou le support inerte ne sont pas solubles dans l'eau et que l'agent vecteur de l'application est l'eau.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, des pigments, des colorants, des polymères.

Plus généralement, les compositions selon l'invention peuvent être associées à tous les additifs solides ou liquides correspondant aux techniques habituelles de la mise en formulation pour application du traitement de semences notamment.

On notera à ce propos que dans le jargon des hommes de métier, le terme traitement de semences se rapporte en fait au traitement des graines.

Les techniques d'application sont bien connues de l'homme de métier et elles peuvent être utilisées sans inconvénient dans le cadre de la présente invention.

On pourra citer par exemple le pelliculage ou l'enrobage.

Parmi les compositions, on peut citer de manière générale les compositions solides ou liquides. Ces compositions sont diluées à l'eau de manière à donner un mélange homogène pouvant être utilisé dans les équipements habituels pour traiter les produits de multiplication notamment les semences.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Les compositions peuvent encore être utilisées sous forme de poudre pour poudrage ; on peut ainsi utiliser une composition comprenant 50 g de matière active, 10 g de silice finement divisée, 10 g de pigment organique et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les granulés dispersables.

Les concentrés émulsionnables ou solubles comprennent le plus souvent 10 à 80 % de matière active, les émulsions ou solutions prêtes à l'application contenant, quant à elles, 0,01 à 20 % de matière active.

Par exemple, en plus du solvant, les concentrés épmulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les semences.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des pigments, des colorants, des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau. Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des pigments, des colorants, des agents de pénétration, des adhésifs, ou des agents antimottants, colorants, etc ... Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la

mouillabilité et la mise en suspension sont avantageuses ; on peut les mettre en suspension avec de l'eau à toute concentration désirée et ces suspensions sont utilisables très avantageusement en particulier pour l'application sur les semences. A la place des poudres mouillables, on peut réaliser des pâtes. Les conditions et modalités de réalisation et d'utilisation de ces pâtes sont semblables à celles des poudres mouillables ou poudres à pulvériser.

Les granulés dispersables sont habituellement préparés par agglomération, dans des systèmes de granulation appropriés, des compositions de type poudre mouillable.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Parmi ces compositions, l'homme de métier choisira avantageusement celle ou celles convenant selon les conditions de mise en oeuvre.

Selon une variante préférée la composition selon l'invention contiendra également un pigment connu en soi pour diminuer la phytotoxicité des triazoles. Cette variante peut être intéressante dans le cas des fortes doses de fongicide, pour les dicotylédones surtout.

Dans le cas du riz, il a été trouvé que la composition avait également un effet désinfectant.

Le procédé selon l'invention peut être utilisé pour la protection tant préventive que curative des produits de multiplication des végétaux contre les champignons, notamment de type basidiomycètes, ascomycètes, adelomycètes ou fungi-imperfecti, en particulier les rouilles, les caries, les oïdium, le piétin-verse, les fusarioses, fusarium roseum, fusarium nivale, helminthosporioses, rhynchosporioses, septorioses, rhizoctones des végétaux et des plantes en général et en particulier des céréales telles que le blé, l'orge, le seigle , l'avoine et leurs hybrides et aussi le riz et le maïs.

Le procédé selon l'invention permet en particulier de lutter contre les champignons notamment de type basidiomycètes, ascomycètes, adelomycètes ou fungi-imperfecti comme Botrytis cinerea, Erysiphe graminis, Puccinia graminis, Puccinia recondita, Piricularia oryzae, Cercospora beticola, Puccinia striiformis, Erysiphe cichoracearum, Rhinchosporium secalis, Fusarium, Solani, Fusarium oxysporum (melonis par exemple), Pyrenophora avenae, Septoria tritici, Septoria avenae, Whetzelinia sclerotiorum, Mycosphaerella fijiensis, Alternaria solani, Aspergillus niger, Cercospora arachidicola, Cladosporium herbarum, Tilletia caries, Tilletia contreversa, Fusarium roseum, Fusarium nivale, Helminthosporium oryzae, Helminthosporium teres, Helminthosporium gramineum, Helminthosporium sativum, Penicillium expansum, Pestalozzia sp, Phoma betae, Phoma foveata, Phoma ligam, Ustilago maydis, Ustilago nuda, Ustilago hordei, Ustilago avenae, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani, Acrostalagmus koningi, les Alternaria, les Colletotrichum, Corticium rolfsii, Diplodia natalensis, Gaeumannomyces graminis, Gibberella fujikuroi, Hormodendron cladosporioides, Myrothecium verrucaria, Paecylomyces varioti, Pellicularia sasakii, Phellinus megaloporus, Sclerotium rolfsii, Stachybotris atra, Trichoderma pseudokoningi, Trichothecium roseum.

L'invention a également pour objet le produit de multiplication des végétaux avec les variantes préférées telles qu'elles ont été définies ci-dessus revêtus et/ou contenant au moins un composé selon l'invention.

Elle a également pour objet le produit de multiplication des végétaux revêtu de la composition fongicide telle que définie ci-dessus avec donc l'agent tensio actif éventuel et le support éventuellement.

Le terme revêtu de et/ou contenant signifie que la matière active se trouve majoritairement à la surface du produit lors de l'application encore qu'une partie plus ou moins significative puisse y pénétrer selon le mode d'application. Quand ledit produit de multiplication est replanté, il absorbe la matière active.

En fait commercialement on peut avancer que la matière active est à la surface la plupart du temps en majorité.

De préférence le produit de multiplication est choisi parmiles graines choisies avantageusement parmi les graines :

– dicotylédones : pois, concombre, soja, melon, coton, tournesol, colza, haricot, lin, betterave
– monocotyledones : blé tendre d'hiver, blé tendre de printemps, blé dur, orge, seigle, avoine, luzerne, maïs, riz.

De préférence les graines sont revêtues à raison de 0,1 à 500 g de matière active par quintal de graine et de préférence 1 à 400 g/quintal.

Une autre variante avantageuse consiste en ce que le produit de multiplication est un tubercule de pomme de terre de préférence revêtu d'une quantité de matière active correspondant au trempage dudit produit dans une solution contenant 0,1 g/l à 100 g/l de matière active.

L'invention comprend également un procédé de lutte efficace contre les maladies notamment foliaires telles que la pourriture grise (Botrytis) et les cercosporioses, et, de ce fait, elles peuvent être appliquées sur des cultures aussi variées que la vigne, les cultures maraichères et l'arboriculture et les cultures tropicales telles que l'arachide, le bananier, le caféier, la noix de pécan et d'autres.

L'invention concerne également une composition fongicide destinée à mettre en oeuvre le procédé selon l'invention.

De préférence, la composition contient 0,5 à 95 % de matière active.

Procédé de préparation

La présente invention concerne également des procédés de préparation des composés selon l'invention.

Dans l'exposé qui va suivre les substituants décrits ci-dessous ont la même signification que ceux indiqués plus haut, sauf indication contraire.

Le procédé dans le cas où $R_3$ est différent de l'atome d'halogène ou du radical $C_1$-$C_6$ alkoxy et $R_4$ est hydrogène consiste à condenser une cetone de formule

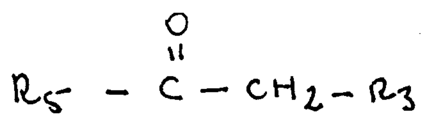

II

avec un aldehyde de formule :

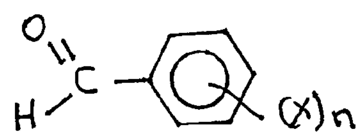

en présence d'une base et, éventuellement d'un solvant, au moyen de la réaction bien connue d'aldolisation crotonisation de façon à obtenir le composé de formule:

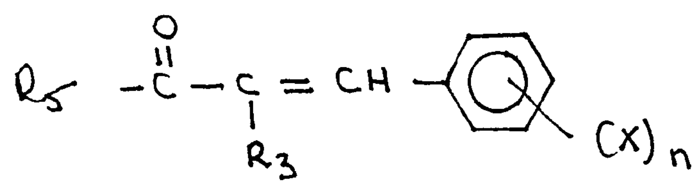

IV

la base pouvant être NAOH, KOH, LIOH, $Ba(OH)_2$ ou diisopropylamidure de lithium, hydrure de sodium, méthylate ou ethylaté de sodium ou diethylamidure de lithium, .le solvant pouvant être un alcool ou un éther (THF, éther éthylique, dioxane)

Les cétones citées ci-dessus de formule IV peuvent également être obtenues par alkylation.

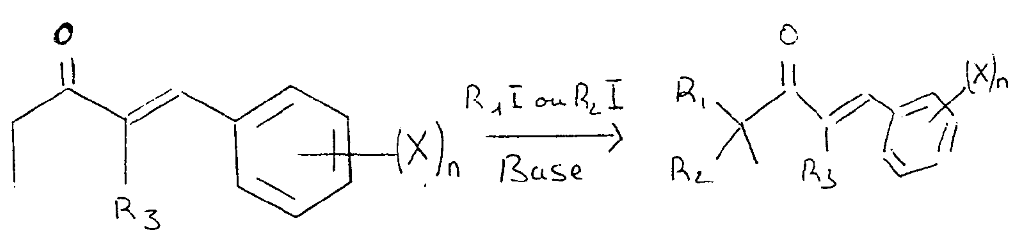


La base pouvant être NAOH, KOH ou un alcoolate de sodium, le solvant étant le toluène, ou le THF ou la NMP.

Ledit composé de formule (IV) est mis à réagir avec un ylure de sulfonium comme décrit dans E.J.COREY M. CHAYKOVSKY, J.Am.Chem. Soc 87, 1313, (1965) afin de conduire aux oxiranes de formule :

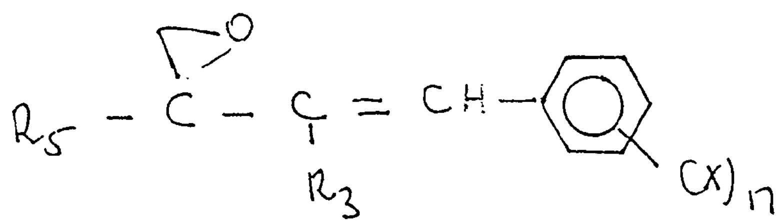

V

On fait réagir ensuite l'oxiranne de formule V avec un imidazole ou un triazole non substitué en présence de base organique ou minérale par exemple la pyridine, la triéthylamine, la soude, la potasse, les carbonates et bicarbonates de métaux alcalins ou alcalino terreux et les hydrures de métaux alcalins et dans un solvant approprié tel que par exemple les alcools, les cétones, les amides, les nitriles, les hydrocarbures aromatiques éventuellement halogénés, à une température comprise entre 80° et le reflux du solvant et dans un rapport molaire V : imidazole ou triazole de préférence compris entre 1,1 et 0,2 ce qui conduit aux composés de formule I annoncé.

Le composé de formule I dans laquelle $R_4$ est différent de l'atome d'hydrogène est obtenu par éthérification des composés de formule I dans laquelle $R_4$ est l'atome d'hydrogène selon des méthodes conventionnelles bien connues de l'homme de l'art : les éthers peuvent être obtenus en traitant un sel alcalin de l'alcool de formule (I) (par exemple un sel de lithium ou de sodium) avec l'halogène approprié de formule $R_4Hal$.

Dans le cas où $R_3$ est un atome d'halogène on prépare les composés de formule (I) en halogénant la cétone alpha-éthylenique de formule IV puis en déshalogénant la cétone dihalogénée au moyen d'une base organique ou minérale comme la soude, la potasse, la triéthylamine dans un solvant aprotique.

La suite de réactions identiques aux précédentes permet d'obtenir les composés de formule (I) annoncés.

Toujours dans le cas où $R_3$ est un atome d'halogène, une autre voie d'accès consiste à partir d'une alphachlorocétone de formule (IX)

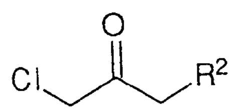

(IX)

et de la faire réagir avec un aldehyde de formule

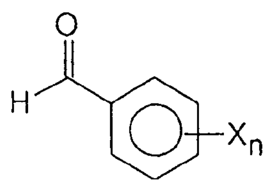

en présence d'un acide tel que l'acide p-toluènesulfonique, acide méthanesulfonique, acide sulfurique dans un solvant tel qu'un hydrocarbure aromatique ou l'acide acétique pour obtenir un composé de formule (X)

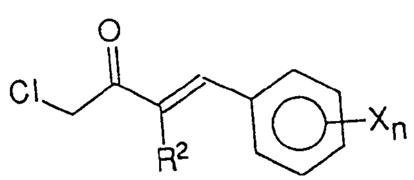

(X)

Ledit composé (X) est mis à réagir avec un organomagnésien $R^1MgX$ (X = Cl, Br) dans un solvant (THF, ether éthylique, hydrocarbures aromatiques) dans un intervalle de température de -20° à +25° C pour obtenir un composé de formule (XI)

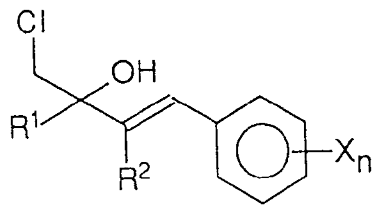

(XI)

Ledit composé (XI) est mis à réagir avec un imidazole ou un triazole en présence d'une base (NaOH, KOH, $Na_2CO_3$, $K_2CO_3$, NaH, DBU, triéthylamine) dans un solvant tel que les alcools $C_1$ à $C_5$, les cétones, les amides, les nitriles, les hydrocarbures aromatique dans une plage de température de 25°C à la tempérture de reflux du solvant, pour obtenir le composé de formule (VIII).

Dans le cas où $R_3$ est $C_1$-$C_6$ alcoxy éventuellement substitué on fait réagir la cétone de formule IV où $R_3$ est un atome d'halogène obtenue au paragraphe précédent avec un alcoolate dans l'alcool correspondant.

Remarques

Les cétones de départ peuvent être obtenues selon

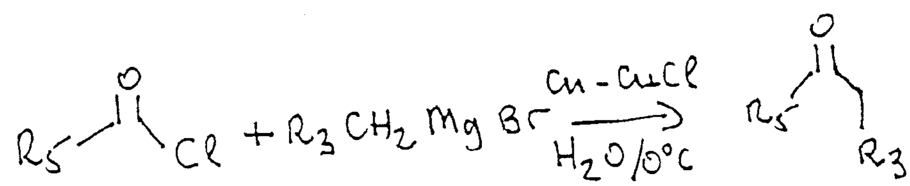


décrite dans la littérature :
(J.E. Dubois, M. Boussu Tetrahedron Lett. 1970, 2523 $R_1$, $R_2$, $R_3$ = H, $C_1$-$C_4$ alkyle, haloalkyle.

Exemple I

Préparation du (E)-1-(4-chlorophenyl)-4,4-dimethyl-3-(1,2,4-triazol-1-ylmethyl)-3-ol-1-pentène.

A un mélange de 136 ml de pinacolone, 141 g de p-chlorobenzaldehyde et 400 ml d'éthanol sont ajoutés 6,6g de potasse en poudre. Lorsque toute la potasse est dissoute, la température du milieu réactionnel est de 50°C. Lorsque la température retombe à 45°C, un solide blanc commence à cristalliser à partir du milieu réactionnel. Après 1h, l'épaisse bouillie est filtrée et les cristaux sont lavés avec 200 ml de méthanol. A partir des eaux mères se déposent encore d'autres cristaux qui sont filtrés et lavés avec 100 ml de methanol. Le solide obtenu est séché sous vide à 50°C 177g de cristaux sont obtenus dans ce premier jet (79 % du rendement théorique). En laissant dans un cristallisoir pendant une nuit, la quantité de cristaux recueillie est portée à 202g. Le composé est le (E)-1-(4-chlorophenyl)-4,4-dimethyl-3-one -1-pentène. (F = 86°).

A un mélange de 50 g de 1-(4-chlorophenyl)-4,4-dimethyl-1-pentène-3-one et 51g de methylsulfate de triméthylsulfonium dans 250 ml de NMP entre O°C et 10°C sont ajoutés 47 g de potasse en poudre. Après 30 mn, 30 g de 1, 2, 4-triazole sont ajoutés et la solution est chauffée à 80°C pendant 2 h. Le mélange est versé dans 1l d'eau et extrait avec 500 ml d'acétate d'éthyle. La phase organique est lavée avec 500 ml d'eau. Après évaporation, 150 ml d'éther diisopropylique sont ajoutés. 16 g de solide blanc (F = 131°C), (composé N° 1) sont obtenus par cristallisation.

Exemple II

Préparation du 1-(4-chlorophényl) 2,4-diméthyl 3-(1,2,4-triazol-1-ylméthyl)-3-ol 1-pentène (composé N° 2)

Un mélange de 158 ml de 3-pentanone, 70,3 g de p-chlorobenzaldehyde, 6,6 g de potasse en poudre et 250 ml d'éthanol est chauffé à reflux pendant 1 h.

Le mélange est ensuite versé dans 250 ml d'eau contenant 5 % d'acide acétique et extrait avec 250 ml de dichlorométhane. La phase organique est séchée sur $NO_2SO_4$ et filtrée. Le solvant et l'excès de 3-pentanone est évaporé. 200 ml de pentane sont ajoutés et 58,8 g de cristaux jaunes sont obtenus (F = 61°C). Le composé est le 2-(4-chlorobenzylidène)-3-pentanone.

Un mélange de 38,7 g de 2-(4-chlorobenzylidène)-3-pentanone, 49 g de potasse en poudre, 46 ml d'iodure de méthyle, 300 ml de toluène est chauffé à reflux pendant 1 h. Le mélange est filtré sur silice et évaporé. Le résidu est distillé sous vide (Eb = 200°C, $5.10^{-2}$ mbar) pour obtenir 18,7 g de liquide qui est le 2-(4-chlorobenzylidène)-4-methyl-3-pentanone.

A un mélange de 18,7 g de 2-(4-chlorobenzylidène)-4-methyl-3-pentanone 19 g de methylsulfate de triméthylsulfonium, 100 ml d'acétonitrile et 2 ml d'eau sont ajoutés 20 g de potasse en poudre. Après 2 h, le mélange est versé dans 200 ml d'eau et 100 ml de dichloromethane. La phase organique est séchée sur $Na_2SO_4$ et évaporée. Le résidu, dissous dans 100 ml de NMP est versé sur 23 g de 1H-1, 2, 4-triazole, 46 g de carbonate de potassium et chauffé à 80°C pendant 5 h. Le mélange est ensuite versé dans 500 ml d'eau et extrait avec 2 x 100 ml d'acétate d'éthyle. La phase organique est lavée avec 200 ml d'eau, puis 200 ml de solution saturée de NaCl et séchée sur $Na_2SO_4$. Après évaporation, le résidu est purifié par chromatographie sur silice (heptane 50-acétate d'éthyle 45-methanol 5). 5,2 g de solide blanc pulvérulent (F = 98°C) est ensuite obtenu par trituration avec de l'éther diisopropylique.

Exemple III

Préparation du 1-(4-chlorophényl) 2,4,4-triméthyl-3-(1,2,4-triazol-1-ylméthyl)-3-one-1-pentène (composé N°1)

A 116 ml de n-butyllithium dans 100 ml de THF à O°C sont ajoutés 29,6 ml de diéthylamine. 30 g de 2,2-dimethyl-3-pentanone dans 50 ml de THF sont ajoutés, suivis de 36,5 g de p-chlorobenzaldehyde dans 100 ml de THF. Lorsque l'addition est finie, la température est ramenée à 25°C. Après 1h, 250 ml d'acide chlorhydrique concentré sont ajoutés et le mélange est agité pendant 1 h. 250 ml de dichlorométhane sont ajoutés. La phase organique est séchée sur $Na_2SO_4$ et évaporée. Le brut est purifié par chromatographique sur silice (heptane 95-acétate d'éthyle 5). 8,5 g de liquide jaune sont obtenus. Ce composé est le 2-(4-chlorobenzylidène)4,4-diméthyl-3-pentanone on fait réagir ce dernier composé comme à l'exemple II par la réaction de Johnson Corey avec le 1H-1, 2, 4-triazole pour obtenir le produit souhaité.

Exemple IV : Préparation du (E)-1-(4-chlorophenyl)-2-éthyl-4-méthyl-3-(1H-1,2,4-triazol-1-ylméthyl)-3-ol-1-pentène (composé 3) (405398)

a) - Acide (E)-alpha-éthyl-4-chlorocinnamique

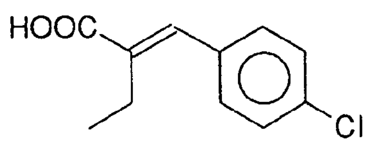

Un mélange de 425 ml d'anhydride butyrique, 220 g de butyrate de sodium, 281 g de 4-chlorobenzaldehyde, 80 gouttes de pyridine est chauffé à 135° C pendant 50 h. 1 l d'eau est ensuite ajouté au milieu réactionnel et 500 ml de soude à 30 %. Lorsque tout le solide est passé en solution, la phase aqueuse est lavée avec 3 X 200 ml d'éther. La phase aqueuse est ensuite acidifiée avec HCl concentré jusqu'à pH3. Le précipité est filtré, lavé à l'eau et séché sous vide à 70° C. 235 g de solide blanc (F = 139° C) sont obtenus (Rdt = 56 %).

b) - (E)-Chlorure d'acide alpha-ethyl-4-chlorocinnamique

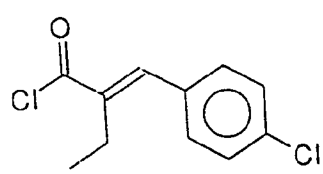

Un mélange de 21 g d'acide alpha-éthyl-4-chlorocinnamique, 40 ml de chlorure de thionyle, 1 ml de N,N-dimethylformamide est chauffé à reflux jusqu'à cessation du dégagement gazeux. L'excès de chlorure de thionyle est éliminé par distillation sous pression réduite. Le résidu est repris avec 100 ml de toluène et évaporé à nouveau. 23 g de produit huileux sont obtenus qui se solidifie sur la paillasse.

c) (E)-1-(4-chlorophenyl)-2-éthyl-4-méthyl-3-one-1-pentène

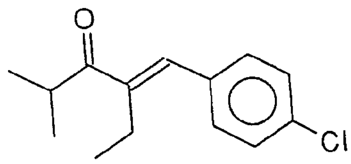

Dans une solution de 23 g de chlorure d'acide alpha-éthyl-4-chlorocinnamique dans 150 ml de tétrahydrofurane (THF) à - 78° C, est ajoutée une solution de chlorure d'isopropylmagnésium (préparée par réaction de 2,5 g de magnésium sur 11 ml de 2-chloropropane dans 50 ml de THF). Lorsque l'addition est finie, le mélange est ramené à température ambiante, versé dans 200 ml d'eau et extrait avec 3 X 100 ml d'éther éthylique. La phase organique est lavée avec une solution aqueuse de NaCl, séchée sur $Na_2SO_4$ et évaporée. 16,4 g d'huile jaune sont obtenue.

d) (E)-1-(4-chlorophenyl)-2-éthyl-4-méthyl-3-(1H-1,2,4-triazol-1-ylméthyl)-3-ol-1-pentène

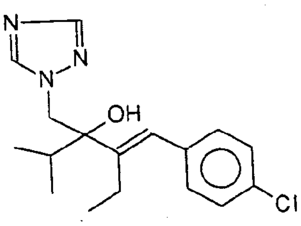

Un mélange de 4 g d'hydrure de sodium, 80 ml de diméthylsulfoxyde et 80 ml d'alcool tert-amylique est chauffé à 40° C. Lorsque la dissolution du solide est terminée, le mélange est refroidi à 0° C et 20,4 g d'iodure de trimethylsulfonium sont ajoutés, suivis de 15,9 g de 1-(-4-chlorophényl)-2-éthyl-4-méthyl-3-one-1-pentène. Après 1 h., 9,9 g de 1H-1,2,4-triazole sont ajoutés avec 19,3 g de carbonate de potassium et le mélange est chauffé à 110° C pendant 8 h. Le mélange réactionnel est ensuite versé dans 500 ml d'eau et extrait avec 3 X 100 ml d'acétate d'éthyle. La phase organique est lavée avec 100 ml d'eau, puis 100 ml de solution aqueuse saturée de NaCl et séchée sur $Na_2SO_4$. Le résidu est purifié par chromatographie sur silice avec heptane 50 - acétate d'éthyle 45 - méthanol 5. 4,3 g de solide blanc (F = 67° C) sont obtenus.

En opérant selon le même mode opératoire avec les réactifs appropriés, on a préparé le 2-(4-chlorobenzylidène)-4,4 dimethyl-3 éthyl 3-(1,2,4-triazol-1-ylméthyl)-3 pentanol de point de fusion 112° C (composé 4) (405149).

Exemple V : Préparation du (Z)-1-(4-Chlorophényl)-2-chloro-3-hydroxy-3-(1H-1,2,4-triazol-1-ylméthyl)-4-méthyl-1-pentène (composé 5)

a) (Z)-1-(4-Chlorophenyl)-2,4-dichloro-3-one-1-butène

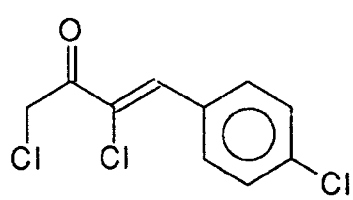

Un mélange de 50 g de 1,3-dichloroacétone, 46 g de 4-chlorobenzaldéhyde, 5,7 g d'acide p-toluènesulfonique et 250 ml de toluène est chauffé à reflux sous atmosphère d'azote dans un Dean-Stark pendant 24 h. La solution est ensuite lavée avec 200 ml d'eau et la phase organique est évaporée. Le résidu est repris avec 200 ml d'éther d'isopropylique et le précipité formé est filtré. 38 g de solide beige (F = 122° C) sont obtenus. Rdt = 46 %.

b) (Z)-1-(4-Chlorophényl)-2-chloro-3-hydroxy-3-chlorométhyl-4-méthyl-1-pentène

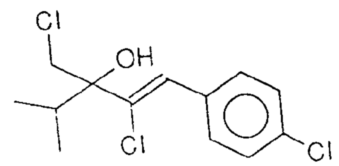

A 2,5 g de magnésium en copeaux dans 10 ml de THF sont ajoutés 8 ml de chlorure d'isopropyle en solution dans 50 ml de THF. Cette solution d'organomagnésien est ensuite transférée sur une solution de 18 g de 1-(-4-chlorophényl)--2,4-dichloro-3-one-1-butène, 18,3 g de perchlorate de lithium dans 50 ml de THF à 0° C. Le mélange est agité 1 h à 0° C. 7 ml d'acide acétique sont ajoutés. Le mélange est versé dans 100 ml d'eau et 100 ml d'acétate d'éthyle. La phase organique est séchée sur sulfate de sodium. Le résidu après évaporation, est purifié par chromatographie sur silice avec heptane 90 - acétate d'éthyle 10. 5,6 g d'huile sont obtenus.

c) (Z)-1-(4-Chlorophényl)-2-chloro-3-hydroxy-3-(1H-1,2,4-triazol-1-ylméthyl)-4-méthyl-1-pentène

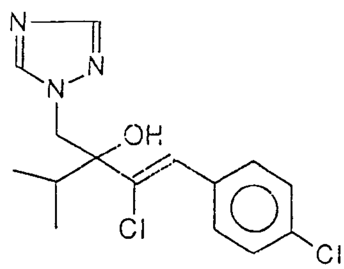

A 20,1 g de 1-(4-chlorophenyl)-2-chloro-3-hydroxy-3-chloromethyl-4-methyl-1-pentène dans 50 ml de N-méthylpyrrolidone (NMP) sont ajoutés 6,9 g de 1H-1,2,4-triazolyl sodium. La température passe de 25° à 60° C. Après 30 mn à 60° C, le mélange est versé dans 100 ml d'eau et 100 ml d'acétate d'éthyle. La phase organique est lavée avec 100 ml de saumure et séchée sur sulfate de sodium. Le résidu après évaporation, est purifié par chromatographie sur silice avec heptane 50 - acétate d'éthyle 45 - méthanol 5. 2,5 g de solide blanc (F = 90° C) sont obtenus.

En opérant selon le même mode opératoire avec les réactifs appropriés, on a préparé le :
2-(4 chlorobenzène)-4 méthyl-3-chloro 3(1,2,4-triazol-1 ylméthyl)-3 pentanol (composé 6) (405503) de point de fusion 90° C.

Exemple VI : Préparation du (Z)-1-(4-chlorophényl)-2-chloro-3-hydroxy-3-(1H-1,2,4-triazol-1-ylméthyl)-4-carboxylate d'éthyle-1-butène (composé N° 7)

a) (Z)-1-(4-chlorophényl)-2-chloro-3-one-4-(1H-1,2,4-triazol-1-yl)-1-butène

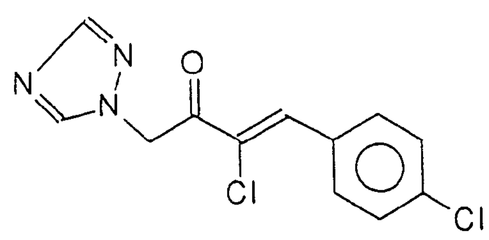

Un mélange de 87,7 g de 1-(4-chlorophényl)-2,4-dichloro-3-one-1-butène comme celui obtenu à l'exemple Va et de 30,9 g de 4-amino-1,2,4-triazole est chauffé à reflux dans 350 ml d'acétonitrile. Après 1 h de reflux, le solide blanc formé est filtré et lavé avec de l'acétonitrile. Les 107 g de solide ainsi obtenus sont mis en suspension dans 200 ml d'eau et 200 ml de dioxane. 63 ml d'acide chlorhydrique concentré sont ajoutés, suivis d'une solution aqueuse saturée de 23,2 g de nitrite de sodium. Après 3 h à température ambiante, le dégagement gazeux a cessé et le mélange est neutralisé jusqu'à pH7 avec une solution saturée de carbonate de potassium. Le mélange est extrait avec de l'acétate d'éthyle. Après évaporation du solvant, 66 g de solide beige sont obtenus.
Rdt = 67 %
F = 141° C

b) (Z)-1-(4-chlorophényl)-2-chloro-3-hydroxy-3-(1H-1,2,4-triazol-1-ylméthyl)-4-carboxylate d'éthyle-1-butène

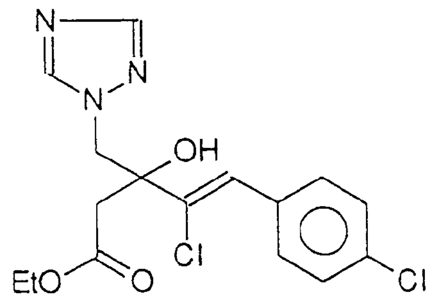

A une suspension de 37 g de zinc dans 250 ml d'éther sont ajoutés 5,1 ml de chlorotriméthylsilane. La suspension est agitée pendant 30 mn à température ambiante et chauffée ensuite 30 mn à reflux. 46,3 ml de bromoacétate d'éthyle sont ajoutés au mélange à reflux à un rythme assez rapide. Le reflux est maintenu pendant 2 h. 250 ml de dichlorométhane sont ensuite ajoutés rendant le mélange plus homogène. 47 g de 1-(4-chlorophényl)-2-chloro-3-one-4-(1H-1,2,4-triazol-1-yl)-1-butène dans 200 ml de dichlorométhane sont ajoutés à température ambiante et la solution est agitée pendant 2 h. Le mélange est versé dans 300 ml d'ammoniaque à 28 %. La phase organique est séchée sur sulfate de sodium et évaporée. Le résidu est recristallisé dans de l'éther diisopropylique. 47,5 g de solide sont obtenus.
Rdt = 76 %
F = 82° C

Exemple VII : (Z)-1-(4-chlorophényl)-2-chloro-3,5-dihydroxy-3-(1H-1,2,4-triazol-1-ylméthyl)-5-méthyl-1-hexène (composé N° 8)

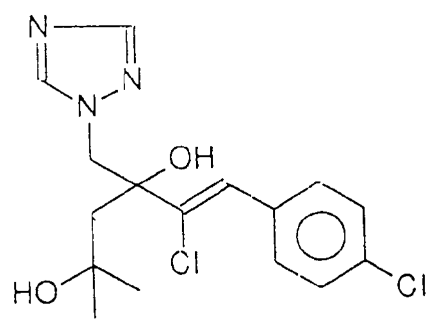

A 2 g de magnésium dans 10 ml d'éther sont ajoutés 5,2 ml d'iodure de méthyle dans 50 ml d'éther. Lorsque le magnésium a entièrement réagi, 10 g de (Z)-1-(4-chlorophényl)-2-chloro-3-hydroxy-3-(1H-1,2,4-triazol-1-ylméthyl)-4-carboxylate d'éthyle-1-butène dans 200 ml de toluène sont ajoutés à 0° C. Le mélange est ensuite chauffé à 50° C pendant 30 mn. La suspension est versée dans un mélange de 100 ml d'eau, 50 ml d'acide acétique et 100 ml d'acétate d'éthyle. La phase organique est lavée avec 100 ml d'eau puis avec 100 ml de solution aqueuse de NaCl, séchée sur sulfate de sodium et évaporée. Le résidu est purifié par chromatographie sur silice avec heptane 50 - acétate d'éthyle 45 - methanol 5. F = 122° C.

Exemple VIII : Test biologique in vivo sur "Puccinia recondita" par application en traitement de semences.

On prépare par broyage fin une émulsion aqueuse de la matière active à tester ayant la composition suivante :

- matière active à tester : 60 mg
- Tween 80 (agent tensio actif) constitué d'un oléate de dérivé polyoxyéthylène du sorbitan) dilué à 10 %

dans l'eau : 0,3 ml

– on complète à 60 ml d'eau.

Cette émulsion aqueuse est ensuite diluée par de l'eau pour obtenir la concentration désirée.

Des graines de blé variété Talent sont traitées avec la bouillie obtenue aux doses de 6, 12, 25, 50, 100, 200 g/q , semées dans un substrat composé d'un mélange tourbe terre pouzzolane 50/50. 15 jours après le semis les plantules sont inoculées par une suspension aqueuse de spores (50000 Sp/cm3) pulvérisée. Cette suspension a été obtenue à partir de plants contaminés.

La lecture des résultats est faite 30 jours et 45 jours après le semis.

Aux doses indiquées avec les composés 1, 2, 3, le pourcentage d'infection est nul 30 jours après le semis, les plantules issues de graines non traitées étant contaminées à 100 %. On observe aucune réduction de taille excepté un faible effet régulateur de croissance à la plus forte dose.

Exemple IX : Test in vivo sur Botrytis cinerea sur feuille excisée de tomate

On prépare par broyage fin une émulsion aqueuse de la matière active à tester ayant la composition suivante :

– matière active à tester : 60 mg

– Tween 80 (agent tensio actif) constitué d'un oléate de dérivé polyoxyéthylène du sorbitan) dilué à 10 % dans l'eau : 0,3 ml

– on complète à 60 ml d'eau.

Cette émulsion aqueuse est ensuite diluée par de l'eau pour obtenir la concentration désirée.

Des tomates cultivées en serre (variété Marmande) âgées de 30 à 40 jours sont traitées par pulvérisation avec des émulsions aqueuses (appelées bouillies) telles que définies ci-dessus et à diverses concentrations du composé à tester. L'essai est répété deux fois avec chaque concentration. Après 24 ou 48 heures, les feuilles sont coupées et mises dans 2 boîtes de Pétri (diamètre 14 cm) dont le fond a été préalablement garni d'un disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (100.000 unités/cm3).

Le contrôle est fait à 3 et 6 jours après la contamination par comparaison avec un témoin non traité.

Dans ces conditions, on observe après 6 jours, à la dose de 1 g/l, une protection bonne ou totale avec les composés 2, 3, 5, 6, 7, 8.

## Revendications

**1)** Procédé pour protéger à titre curatif ou préventif les produits de multiplications des végétaux et les végétaux en résultant contre les maladies fongiques, caractérisé en ce que l'on applique audit produit de multiplications une composition fongicide contenant un support inerte acceptable en agriculture, éventuellement un tensio actif également acceptable en agriculture et au moins un azole de formule :

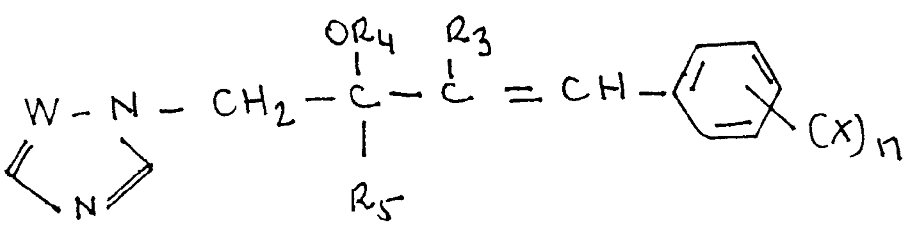

I

dans laquelle :

– W représente un groupe trivalent constitué soit d'un groupe = CH-, soit d'un atome d'azote -N=,

– X est un atome d'halogène, notamment le chlore, le brome, le fluor, ou un groupe $C_1$-$C_4$ alkyle ou $C_1$-$C_4$ alkoxy éventuellement halogéné.

n est un nombre entier positif ou nul, inférieur à 6, les groupements X pouvant être identiques ou différents lorsque n est plus grand que 1,

$R_1$, $R_2$, identiques ou différents, représentent l'atome d'hydrogène ou un radical $C_1$-$C_4$ alkyle ou alkylène

$C_2$-$C_4$ éventuellement substitué (par exemple par un ou plusieurs atomes ou radicaux tels que les atomes d'halogènes, les radicaux $C_1$-$C_4$ alkoxy), les radicaux $C_3$-$C_7$ cycloalkyle, $C_6$-$C_{10}$ aryle (notamment phényle), $C_7$-$C_{11}$ aralkyle (notamment benzyle), ces divers radicaux pouvant être éventuellement substitués (par exemple par un ou plusieurs atomes ou radicaux tels que les atomes d'halogènes, les radicaux $C_1$-$C_4$ alkyle, les radicaux mono ou polyhalo $C_1$-$C_4$ alkyle, les radicaux $C_1$-$C_4$ alkoxy et les radicaux mono ou polyhalo $C_1$-$C_4$ alkoxy), $R_1$, $R_2$ ensemble peuvent former une chaine $C_2$-$C_5$ hydrocarbonée constituant un cycle avec le carbone auquel $R_1$, $R_2$ sont reliés, cette chaine étant éventuellement substituée comme pour les radicaux $C_6$-$C_{10}$ aryle précédemment cités

$R_4$ représente l'atome d'hydrogène, un radical $C_1$-$C_4$ alkyle éventuellement substitué (par exemple par un ou plusieurs atomes ou radicaux tels que les atomes d'halogènes, les radicaux $C_1$-$C_4$ alkoxy,

$R_5$ est un groupe $C(R_1,R_2)CH_3$ dans lequel $R_1$ et $R_2$ sont tels que définis ci-dessus, ou un groupe $CH_2C(R_6,R_7)$-$OR_8$ dans lequel $R_6$ et $R_7$, identiques ou différents, sont un atome d'hydrogène ou un méthyle ou peuvent former ensemble un = O et $R_8$ est un atome d'hydrogène ou un alcoyle de 1 à 3 atomes de carbone.

$R_3$ est un atome d'hydrogène ou un radical $C_1$-$C_6$ alkyle éventuellement substitué (par exemple par un ou plusieurs atomes ou radicaux tels que les atomes d'halogène, les radicaux $C_1$-$C_4$ alkoxy) ou $C_3$-$C_7$ cycloalkyle éventuellement substitué comme pour les radicaux $R_1$ ou $R_2$ ci-dessus,

$R_3$ est encore un radical $C_1$-$C_6$ alcoxy substitué éventuellement comme le radical $C_1$-$C_6$ alkyle ou un atome d'halogène (de préférence F, C1, Br) et les sels acceptables en agriculture de ces composés.

**2)** Procédé selon la revendication 1, caractérisé en ce que dans la formule 1, $R_5$ est le groupe $C(R_1, R_2)CH_3$.

**3)** Procédé selon la revendication 1, caractérisé en ce que dans le composé de formule I, n = 1,2 ou 3.

**4)** Procédé selon la revendication 3, caractérisé en ce que X est un atome d'halogène choisi parmi le chlore, le brome, le fluor.

**5)** Procédé selon la revendication 4, caractérisé en ce que n = 1 ou 2 et X est placé en para lorsque n = 1 et meta para lorsque n = 2.

**6)** Procédé selon la revendication 5, caractérisé en ce que n = 1.

**7)** Procédé selon l'une des revendications 4, 5 ou 6, caractérisé en ce que X est l'atome de chlore.

**8)** Procédé selon la revendication 1, caractérisé en ce que dans le composé de formule I, W est l'atome d'azote.

**9)** Procédé selon la revendication 1, caractérisé en ce que dans le composé de formule I, $R_1$, $R_2$ sont choisis parmi l'atome d'hydrogène ou les radicaux $C_1$-$C_4$ alkyle, de préférence méthyle ou éthyle.

**10)** Procédé selon la revendication 1, caractérisé en ce que dans le composé de formule I, $R_4$ est l'atome d'hydrogène ou un radical $C_1$-$C_4$ alkyle, de préférence l'atome d'hydrogène.

**11)** Procédé selon la revendication 1, caractérisé en ce que dans le composé de formule I, $R_3$ est un atome d'hydrogène ou de chlore ou un radical $C_1$-$C_4$ alkyle, de préférence méthyle ou éthyle.

**12)** Procédé selon l'une des revendications 1 à 11, caractérisé en ce que la composition contient 0,5 à 95% de composé de formule I.

**13)** Procédé de lutte contre les maladies foliaires des plantes caractérisé en ce qu'on applique un composé de formule I

**14)** Produit de multiplication des végétaux recouvert d'une composition telle que définie aux revendications 1 à 12, ou d'un composé de formule I tel que défini aux revendications 1 à 10.

**15)** Composés azoliques de formule I tels que définis aux revendications 1 à 11, à l'exception des composés suivants :

| 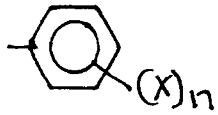 | $R_1$ | $R_2$ | $R_3$ | $R_4$ | W |
|---|---|---|---|---|---|
| 2,4-dichlorophenyl | $CH_3$ | $CH_3$ | H | H | N |
| 4-chlorophenyl | $CH_3$ | $CH_3$ | H | H | N |
| 4-chlorophenyl | $CH_3$ | $CH_3$ | H | H | CH |
| 4-fluorophenyl | $CH_3$ | $CH_3$ | H | H | N |
| 2-chlorophenyl | $CH_3$ | $CH_3$ | H | H | N |
| 2-chloro4-fluorophenyl | $CH_3$ | $CH_3$ | H | H | N |
| 2-fluoro-4chlorophenyl | $CH_3$ | $CH_3$ | H | H | N |
| 4-chlorophenyl | -1-methylcyclopropyl- | | H | H | N |
| 4-methoxyphenyl | $CH_3$ | $CH_3$ | H | H | N |
| 4-methylphenyl | $CH_3$ | $CH_3$ | H | H | N |
| 4-chlorophenyl | $CH_3$ | $CH_3$ | H | $CH_3$ | N |
| 4-fluorophényl | $CH_3$ | $CH_3$ | H | $CH_3$ | N |
| phenyl | $CH_3$ | $CH_3$ | H | H | N |
| 4-methoxyphenyl | $CH_3$ | $CH_3$ | H | $CH_3$ | N |
| 2-methylphenyl | $CH_3$ | $CH_3$ | H | H | N |
| 2-methylphenyl | $CH_3$ | $CH_3$ | H | H | CH |
| 4-fluorophenyl | $CH_3$ | $CH_3$ | H | H | CH |
| 4-methylphenyl | $CH_3$ | $CH_3$ | H | H | CH |
| 2,6-dichlorophenyl | $CH_3$ | $CH_3$ | H | H | CH |

**16)** Compositions fongicides caractérisées en ce qu'elles contiennent comme matière active au moins un composé selon la revendication 15.

Office européen des brevets

# RAPPORT DE RECHERCHE EUROPEENNE

Numero de la demande

EP 91 42 0400

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,X | EP-A-0 040 345 (BAYER AG) *pages 17, 19, 21, 24* * page 36, ligne 6 - page 37, ligne 4; revendications; exemples E,F * | 1-16 | A01N43/653<br>A01N43/50<br>C07D249/08<br>C07D233/60<br>C07D521/00 |
| X | EP-A-0 072 580 (BAYER AG) *pages 7, 9, 11, 13* * page 25, ligne 17 - page 26, ligne 14; revendications; exemples E,F * | 1-16 | |
| X | EP-A-0 301 370 (BASF AKTIENGESELLSCHAFT) *pages 6, 7* * page 9, ligne 28 - page 9, ligne 43; revendications * | 1-16 | |
| D,X | EP-A-0 052 424 (IMPERIAL CHEMICAL INDUSTRIES PLC ) * page 15, ligne 1 - page 18, ligne 12; revendications 11,12; exemple 3 * | 1-14 | |
| D,X | EP-A-0 333 059 (BASF AKTIENGESELLSCHAFT) * page 9, ligne 13 - page 9, ligne 56; revendication 5 * | 1-14 | **DOMAINES TECHNIQUES RECHERCHES (Int. Cl.5 )**<br>A01N<br>C07D |

Le présent rapport a été établi pour toutes les revendications

| Lieu de la recherche | Date d'achèvement de la recherche | Examinateur |
|---|---|---|
| LA HAYE | 17 FEVRIER 1992 | DONOVAN T.M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)